# EUROPEAN PATENT SPECIFICATION

(11) **EP 4 153 151 B1**
(45) Date of publication and mention of the grant of the patent: **22.11.2023**
(21) Application number: 21726205.4
(22) Date of filing: 18.05.2021
(51) Int. Cl.: A61K 9/70, A61K 47/10, A61K 47/14, A61K 47/32, A61K 31/196, A61P 19/02, A61P 21/00, A61P 29/00

(54) **SLOW-RELEASE MEDICAL PLASTER**
MEDIZINISCHES PFLASTER MIT LANGSAMER FREISETZUNG
EMPLÂTRE MÉDICAL À LIBÉRATION LENTE

(30) Priority: 20.05.2020 IT 202000011686
(43) Date of publication of application: 29.03.2023
(73) Proprietor: Fidia Farmaceutici S.p.A., 35031 Abano Terme (PD) (IT)
(72) Inventor: PIZZOCARO, Carlo, 35031 Abano Terme (PD) (IT)
(74) Representative: De Gregori, Antonella
(86) International application number: PCT/IB2021/054261
(87) International publication number: WO 2021/234562

(56) References cited:
- EP-A1- 0 950 408
- WO-A1-2012/089256
- WO-A1-2012/160125
- CN-A- 1 489 996
- JP-A- H09 208 463
- US-A1- 2015 202 171
- FINI ADAMO ET AL: "Diclofenac Salts, VIII. Effect of the Counterions on the Permeation through Porcine Membrane from Aqueous Saturated Solutions", PHARMACEUTICS, vol. 4, no. 3, 12 September 2012 (2012-09-12), pages 413-429, XP055822027, CH ISSN: 1999-4923, DOI: 10.3390/pharmaceutics4030413
- Güngör Sevgi ET AL: "Plasticizers in Transdermal Drug Delivery Systems" In: "Recent Advances in Plasticizers", 21 March 2012 (2012-03-21), InTech, XP055820957, ISBN: 978-953-51-0363-9 DOI: 10.5772/38156, the whole document

## Description

### OBJECT OF THE INVENTION

The present invention describes a slow-release medical plaster based on diclofenac, specifically a medical plaster based on diclofenac sodium salt, whose formulation allows the release of the active ingredient continuously and at locally therapeutically active concentrations for 24 hours, and also a polymeric adhesive matrix (PSA) for use in a medical plaster based on diclofenac sodium salt.

The plaster is soft, flexible, it adheres perfectly to the skin, does not come off even when applied to a joint area, it can be removed without causing pain or irritation. These features, together with the fact that the plaster must be replaced only once every 24 hours, make it particularly pleasing for the patient.

### STATE OF ART

The possibility of administering drugs through the skin has been known for some time and is particularly interesting as it is a non-invasive means of administration, metabolically undemanding for the body and pleasanter for the patient than other administration routes. Patients can in fact find relief from localized pain without necessarily taking oral or injected drugs. Over time, numerous adhesive release systems have been developed which, when applied to the skin, release the active ingredient. These systems generally consist of a polymeric adhesive matrix, called "Pressure Sensitive Adhesive" (PSA), which contains, in dispersion or solution, the active ingredient of interest. The matrix is in turn spread on a support and coated with a protective layer, to be removed before application. The polymeric matrix can be of various kinds but generally acrylic and methacrylic polymers and their copolymers are used.

Within the generic category of adhesive release systems described above, transdermal patches and medical plasters can be distinguished, defined in international pharmacopoeias as distinct pharmaceutical forms.

Transdermal patches (Transdermal Patches, European Pharmacopoeia 5.0, 616) are defined as flexible pharmaceutical preparations for topical application, having various shapes and sizes, containing one or more active ingredients, to be applied on healthy skin: the active ingredient is gradually released and, after passing through the skin, it reaches the bloodstream in a therapeutically active quantity. Transdermal patches therefore represent an administration route destined for giving a systemic effect and, in fact, by way of example, transdermal patches based on nicotine for smoking cessation, patches based on sex hormones, usually estrogens, for the climacteric syndrome or opiate-based drugs for the treatment of pain in terminally ill patients, are known in the state of the art.

As already mentioned, these are patches that introduce the drug into the bloodstream in a therapeutically active quantity, so that it reaches the specific site of action. Their use is essentially an alternative to the more classic systemic administration routes, such as oral and injective.

Medical plasters, on the other hand, are another thing which, according to definition (*Medical Plasters,* European Pharmacopoeia 5.0, 626), are flexible preparations for topical application, containing one or more active ingredients, to be applied on the skin, formulated so as to maintain the active ingredient in close contact with the skin, so that it is released slowly while remaining concentrated in the area of application.

In this case, therefore, the active ingredient does not enter the bloodstream except in completely minimal and non-therapeutically significant traces, and acts exclusively in the area in which it was released. Medical plasters therefore well define a topical administration route with local action and are therefore extremely useful in the treatment, even chronic, of localized joint or muscle pain; they act in fact where needed, without passing into the bloodstream, and avoid the systemic administration of drugs with a moderate degree of toxicity, such as non-steroidal anti-inflammatory drugs (NSAIDs), electively used in the above-mentioned diseases.

Numerous medical plasters for topical use based on NSAIDs are described in the state of the art, in particular diclofenac which, as is known, is an acidic molecule and poorly soluble in water. The formulability of diclofenac, when it is to be administered by skin application, is complex: it must be ensured that it penetrates through the skin in sufficient quantities for maintaining therapeutic concentration levels at the site of administration.

The active ingredient must therefore pass through the corneum stratum, which is a hydrophobic barrier, and be diffused in the underlying tissue, characterized by structures and compartments of both a hydrophobic and hydrophilic nature. Diclofenac in its acid, non-dissociated form, has an extremely high partition coefficient: the partition coefficient is the ratio of concentrations of a compound in a mixture of two immiscible solvents (hydrophobic/hydrophilic) at equilibrium. It therefore expresses the degree of hydrophobicity of a compound: the higher this coefficient, the higher the hydrophobicity of the compound. This means that diclofenac in acidic form, by virtue of its partition coefficient, is capable of easily passing through the corneum stratum of the skin, being highly similar to it, but, due to its low solubility in water, it is not diffused with the same ease in the underlying tissue. It is, on the other hand, capable of diffusing in the underlying tissue when it is in its salified, less hydrophobic form, bearing in mind however that the degree of hydrophobicity is naturally related to the type of salt considered. This explains why in general, products for topical application based on diclofenac are formulated with variously salified diclofenac, regardless of the PSA matrix used. Salification in fact attenuates the hydrophobic nature of the molecule, giving it a biphasic character that mimics the biphasic nature of the skin in its entirety, thus ensuring convenient penetration and above all allowing adequate formulability.

For the purposes of the present invention, the following should be remembered:
US2015/202171 A1 addresses the problem of providing an improved patch of diclofenac sodium with an enhanced skin permeability. It discloses a patch comprising an adhesive layer, which comprises 3%wt or more of diclofenac sodium, 3-10%wt of dimethyl sulfoxide and 0.15-9%wt, preferably 0.25-5%wt, of citric acid. With the combined use of dimethyl sulfoxide and citric acid, the crystallization of the drug is suppressed, the skin irritation is lower and the skin permeability is improved. The adhesive layer may also contain a plasticizer, preferably liquid paraffin and liquid polybutene, in an amount of 7 to 70%wt. more preferably of 10 to 60%wt, and an antioxidant, such as butylhydroxyanisole. Co-polymers of methyl (meth)acrylate are cited in a list of adhesives of choice for the adhesive layer.
WO2006097149 which describes a transdermal patch whose PSA matrix based on Eudragit^{®} NE40 (poly(ethyl-acrylate, methyl-methacrylate) 2:1) releases various active ingredients into the bloodstream and, especially when containing some active ingredients, in particular oxybutynin, eliminates the problem of shrinkage of the matrix itself during the preparation phases of the patch;
WO2016059583 which describes a medical plaster whose PSA matrix is composed of DuroTak^{®} 387-2516/87-2516 (acryl-vinyl acetate copolymer, CAS 326602-88-4) and Eudragit^{®}E100 (poly(butyl-methacrylate, (2-dimethylaminoethyl)methacrylate, methyl-methacrylate) 1:2:1), in specific proportions (50-60%: 6-16%); this mixture has proved to ensure high adhesiveness to the plaster, excellent stability and adequate release of the active ingredient dispersed therein, in particular diclofenac, in the form of sodium salt, hydroxyethylpyrrolidine salt (also called epolamine) or diethylammonium salt (DEA) . The concentration of diclofenac present in the plaster, regardless of the type of salification, ranges from 8 to 20% and is on average slightly higher than 15%. The matrix thus formulated is however strongly hydrophobic and therefore significantly slows down the complete release of the active ingredient;
WO2012089256 which describes a medical plaster consisting of a PSA matrix of Eudragit^{®} NE40 (poly(ethyl-acrylate, methyl-methacrylate) 2:1) at 30-55% and an ester of citric acid at 42-55% as plasticizer, in which diclofenac diethylammonium salt (DEA) is dispersed, which is released continuously over 24 hours. The choice of diclofenac salt is not accidental as it has been demonstrated by the inventors themselves that, with the same composition in terms of matrix, active ingredient and excipients, diclofenac DEA salt is released in a constant, continuous, prolonged and therapeutically effective way for 24 hours; diclofenac sodium salt, on the contrary, is released in a minimal way, absolutely unsuitable for exerting the desired pharmacological effect, either for 24 hours or for shorter time intervals. Basically, it follows that the sodium salt of diclofenac is absolutely unsuitable for being included in a medical plaster whose PSA matrix is based on Eudragit^{®} NE40 in formulation with a citric acid ester.

The objective of the present invention is to overcome the drawbacks and problems previously indicated that characterize the state of the art.

The Applicant has in fact surprisingly found that, starting from a PSA matrix of Ethylacrylate-MethylMethacrylate mixed with suitable excipients, it is possible to prepare a medical plaster capable of releasing diclofenac sodium salt in a constant, continuous, prolonged manner, at locally therapeutically active doses for 24 hours. The release of diclofenac sodium salt has a profile similar to that of a market leading product, as will be demonstrated hereunder.

This result, which is achieved by adding to the matrix a component normally used as an antioxidant but never used for the purposes described herein, represents a clear overcoming of the state of the art: an easily formulated long-lasting medical plaster is in fact made available, practically free of toxicity and side-effects and pleasing for the patient, who can replace it only once in 24 hours.

### DESCRIPTION OF THE INVENTION

The present invention relates to a medical plaster comprising
- a base layer (backing),
- a "Pressure Sensitive Adhesive" (PSA) matrix,
- a protective coating layer (liner),

wherein the PSA matrix comprises
   - a neutral copolymer based on ethyl acrylate and methyl methacrylate in a 2:1 ratio, in a concentration ranging from 40 to 49% by dry weight with respect to the dry weight of the matrix;
   - a plasticizing agent selected from esters of citric acid, in a concentration ranging from 40 to 49% by weight with respect to the dry weight of the matrix;
   - butylhydroxyanisole (BHA) in a concentration ranging from 0.10 to 0.20% by weight with respect to the dry weight of the matrix;
there being dispersed in said PSA matrix
   - an active ingredient, which is diclofenac sodium, in a concentration ranging from 1 to 20% by weight with respect to the dry weight of the matrix.

It is specified that the neutral copolymer based on ethyl acrylate and methyl methacrylate in a 2:1 ratio is available on the market in an aqueous dispersion at 30% or 40% by weight of dry product (respectively Eudragit^{®} NE30D and Eudragit^{®} NE40D, Evonik Industries AG Technical Information).

According to the present invention, the Applicant claims a medical plaster comprising a PSA matrix, and the PSA matrix itself, comprising
- a neutral copolymer based on ethyl acrylate and methyl methacrylate in a 2:1 ratio, in a concentration ranging from 40 to 49% by dry weight with respect to the dry weight of the matrix
the range from 40 to 49% by dry weight of said copolymer thus referring to the weight of the dry product contained in the aqueous dispersion as above disclosed.

It is preferred the neutral copolymer based on ethyl acrylate and methyl methacrylate in a 2:1 ratio, in an aqueous dispersion at 40% by weight (Eudragit^{®} NE40D).

In the medical plaster according to the present invention, the "Pressure Sensitive Adhesive" (PSA) matrix is applied by spreading on the base layer (backing), said matrix then being coated by the protective coating layer (liner), to be removed before application.

The main advantage of the medical plaster containing diclofenac sodium salt according to the present invention consists in the fact that, after local application, it releases the active ingredient for the following 24 hours at therapeutically active concentrations on the site of application. The plaster is soft, flexible, adheres perfectly to the skin, is removed without causing pain or irritation, maintains the stability of the active ingredient over time and, ensuring its pharmacological effect for 24 hours, significantly improves the compliance of the patient, who will have to replace the plaster only once, and not two or more, times a day.

The present invention further relates to said medical plaster for use in the once-a-day treatment of painful and inflammatory conditions affecting the musculoskeletal system, such as for example osteoarthritis, and also traumas such as sprains, muscle tears, bruises with intact skin, more specifically when a localized effect is desired and it is preferable or necessary to avoid the administration of painkillers/anti-inflammatory drugs orally or by injection.

The present invention also relates to a "Pressure Sensitive Adhesive" (PSA) matrix comprising or consisting of:
- a neutral copolymer based on ethyl acrylate and methyl methacrylate in a 2:1 ratio, in a concentration ranging from 40 to 49% by dry weight with respect to the dry weight of the matrix;
- a plasticizing agent selected from esters of citric acid, in a concentration ranging from 40 to 49% by weight with respect to the dry weight of the matrix, as above defined;
- butylhydroxyanisole (BHA) in a concentration ranging from 0.10 to 0.20% by weight with respect to the dry weight of the matrix;
   there being dispersed in said PSA matrix
- an active ingredient, which is diclofenac sodium, in a concentration ranging from 1 to 20% by weight with respect to the dry weight of the matrix.

The medical plaster according to the present invention comprises or consists in its entirety of:
- a layer that acts as a base (backing), on which it is uniformly spread
- a "Pressure Sensitive Adhesive" matrix in which it is dispersed
- the active ingredient, this matrix being coated by
- a protective layer that acts as a liner, to be removed before application.

The backing is made of a soft and flexible material, shapeable in any form and size: in general a polymer base is used, in particular polyester is used. In the plaster described herein, the backing is a non-perforated 100% polyester non-woven fabric, which improves adhesion to the skin and prevents the adhesive matrix, and with it the dispersed drug, from leaking out.

The liner is a protective sheet of monosilicone paper, which can be easily removed.

The active principle used in the medical plaster according to the present invention is diclofenac sodium salt (DicloNa); as already mentioned, the choice of salifying diclofenac is necessary, considering the chemical-physical properties of this active ingredient. With respect to the choice of salt, it is known in the state of the art that the nature of the counter-ion with which diclofenac is salified is extremely important: in particular, it is essential for the salt to maintain sufficient hydrophobicity as to be able to pass through the corneum stratum of the skin but to be sufficiently hydrophilic as to be able to be diffused in the underlying layers, reaching locally therapeutically active concentrations, as required for a medical plaster. The diclofenac salts that have shown the best performance in this sense are organic salts with aliphatic amines, both linear and cyclic. Diclofenac salts with inorganic bases, in particular with potassium or sodium, on the contrary, are less suitable for this type of application as they have very low partition coefficients, up to 3,000 times lower than those of acid diclofenac; their very high hydrophilicity therefore strongly limits their passage through the corneum stratum of the skin (Fini et al., 2012, Pharmaceutics, 4, 413-429).

In confirmation of this, there are products on the market based on diclofenac for topical application in the form of emulgel, in which the active ingredient is salified with diethylamine, which is a linear amine (e.g., Voltaren Emulgel^{®}), or in the form of a medical plaster, more interesting for the purposes of the present invention, in which the salification takes place with hydroxyethylpyrrolidine, which is a cyclic amine (for example the medical plaster Flector^{®}).

The possibility of using the sodium salt of diclofenac represents a marked improvement with respect to the state of the art in terms of industrial processing and potential side-effects of the final product. With respect to the industrial aspect, the synthesis of the sodium salt is effected very simply and economically, without the use of toxic reagents, without the need for the controlled disposal of processing waste. As regards the potential side-effects of the final product, it is known that aliphatic amines, especially cyclic amines, can be toxic or at least very frequently give rise to irritative phenomena, due to the release of the amine as such or of its metabolites (Myers et al, 1997, J Tox Subst Mech, 16, 2; Greim et al., 1998, Chemosphere, 36, 271-295).

The use of the sodium salt of diclofenac therefore eliminates these problems and makes the plaster object of the invention also applicable to sensitive and easily irritable skin, such as that, for example, of an elderly patient or potentially allergic or atopic subjects.

The PSA matrix is composed of an aqueous dispersion of copolymers deriving from esters of acrylic and methacrylic acids, preferably it is composed of Eudragit^{®} NE40D (Evonik Industries), i.e. a poly(ethyl-acrylate, methylmethacrylate) copolymer in a ratio of 2:1 in an aqueous dispersion at 40% by weight. A plasticizing agent selected from esters of citric acid is added to this copolymer, preferably a plasticizing agent which is tributyl citrate; a matrix similar to that described for example in WO2012089256 is thus obtained, already tested, unsuccessfully, for the release of diclofenac in the form of sodium salt.

The Applicant has however surprisingly discovered that the addition to the matrix described above of minimal quantities of butylhydroxyanisole (BHA) significantly modifies its properties, allowing the DicloNa dispersed therein to be released in a constant, continuous, prolonged manner, at locally therapeutically active doses for a duration of 24 hours.

The release profile is completely comparable to that of a commercial product widely used as a once-a-day plaster (Flector^{®}), containing diclofenac salt of hydroxyethylpyrrolidine, therefore a salt with a cyclic amine, and a completely different PSA matrix (based on gelatin, polyvinylpyrrolidone, carboxymethylcellulose and other polymers).

BHA is an ingredient with an antioxidant and preservative action, widely used in the food, cosmetic, and animal feed industries for avoiding rancidity of the fats contained therein. It is also used in pharmaceuticals, as an excipient in solid formulations of active ingredients of a lipid nature, specifically for avoiding oxidation and therefore the loss, or at least the reduction, of pharmacological activity: they are formulated with BHA, for example, isotretinoin, some statins and ketoconazole (Braz J Pharm Sci, 2012, 48, 405-4015). BHA is also used in liquid pharmaceutical formulations, for example, injections, in the presence of unstable active ingredients.

Within the scope of the present invention, BHA is used for the first time in a solid composition for topical use devoid of lipid components and which therefore does not require an antioxidant, thus ensuring that, as will be demonstrated hereunder, the diclofenac sodium dispersed in the PSA matrix of the medical plaster object of the invention is released in a constant and continuous manner over 24 hours, possibly reaching the circulatory stream only in traces, thus respecting the definition of medical plaster.

This result is completely contrary and unexpected with respect to the teachings of the state of the art.

The evaluation of the release of diclofenac is effected through the Dissolution Test described in the Official Pharmacopoeias, more specifically in the present application through the Dissolution Test described in the European Pharmacopoeia (Ph. Eur. 5.0, 2.9.4). In relation to the different pharmaceutical forms to be tested, the devices for carrying out the Dissolution Test differ from each other in the type of support in which the material to be analyzed is placed. The general methods for carrying out the test are the same: briefly, the sample to be analyzed is positioned in a suitable support, in turn inserted in a larger container in which a medium (Dissolution Medium) heated to a specific temperature is placed, which comes into contact with the sample. The drug is progressively transferred from the sample to the dissolution medium; at regular intervals fractions of the dissolution medium are collected and analyzed in order to calculate the quantity of drug present therein. The analysis is generally effected with a spectroscope or with high-pressure liquid chromatography (HPLC), more frequently with HPLC. In the Dissolution Test it is essential to operate under sink condition, i.e. with large volumes of dissolution medium: only in this way it is possible, in fact, to avoid that the extent of dissolution of the active ingredient be lower than the actual dissolution, due to the progressive increase in concentration in the receiving medium which, if in small volumes, rapidly approaches saturation.

The PSA matrix of the medical plaster according to the present invention comprises or consists of:
- a neutral copolymer based on ethyl acrylate and methyl methacrylate in a 2:1 ratio, in a concentration ranging from 40 to 49% by dry weight with respect to the dry weight of the matrix, as defined above;
- a plasticizing agent selected from esters of citric acid, in a concentration ranging from 40 to 49% by weight with respect to the dry weight of the matrix;
- butylhydroxyanisole (BHA) in a concentration ranging from 0.10 to 0.20% by weight with respect to the dry weight of the matrix;
   there being dispersed in said PSA matrix
- an active ingredient, which is diclofenac sodium, in a concentration ranging from 1 to 20% by weight with respect to the dry weight of the matrix.

Preferably the esters of citric acid are selected from triethyl citrate, acetyl triethyl citrate, acetyl tributyl citrate, tributyl citrate, more preferably the ester is tributyl citrate.

The composition of the PSA matrix of the medical plaster according to the present invention preferably comprises or consists of:
- a neutral copolymer based on ethyl acrylate and methyl methacrylate in a 2:1 ratio in an aqueous dispersion at 40% by weight, in a concentration ranging from 45 to 48% by dry weight with respect to the dry weight of the matrix, as above defined;
- the plasticizing agent is tributyl citrate, in a concentration ranging from 45 to 48% by weight with respect to the dry weight of the matrix;
- BHA in a concentration ranging from 0.13 to 0.18% by weight with respect to the dry weight of the matrix;
diclofenac sodium being dispersed in said PSA matrix in a concentration ranging from 5 to 10% by weight with respect to the dry weight of the matrix.

The concentrations are always expressed by weight in relation to the dry weight of the matrix; as the pharmacological effect is exerted by diclofenac sodium alone dispersed in the PSA matrix, as the concentration of the latter varies in relation to the amount of active ingredient to be inserted in the plaster, the quantities of the copolymer and plasticizer will consequently also vary, to complete 100% of the final composition.

The composition of the PSA matrix of the medical plaster according to the present invention even more preferably comprises or consists of:
- a neutral copolymer based on ethyl acrylate and methyl methacrylate in a 2:1 ratio in an aqueous dispersion at 40% by weight, in a concentration equal to 46.3% by dry weight with respect to the dry weight of the matrix, as above defined;
- tributyl citrate, in a concentration equal to 46.3% by weight with respect to the dry weight of the matrix;
- BHA in a concentration equal to 0.15% by weight with respect to the dry weight of the matrix;
diclofenac sodium being dispersed in said PSA matrix in a concentration equal to 7.25% by weight with respect to the dry weight of the matrix.

The process for the preparation of the medical plaster object of the invention, containing diclofenac sodium (DicloNa) dispersed in the PSA matrix described above, consists of various phases which can be summarized as follows:
- mixing the plasticising agent selected and BHA until dissolved;
- addition of DicloNa and mixing until dissolved;
- addition of the selected neutral copolymer based on ethyl acrylate and methyl methacrylate in a 2:1 ratio in an aqueous dispersion and mixing until total dispersion;
- pouring the PSA/DicloNa matrix mass on a temporary liner;
- drying of the mass and its coupling with the backing;
- replacement of the provisional liner with the definitive liner;
- cutting the plasters to the desired size;
- insertion of each plaster in its primary packaging.

Some examples are provided for better illustrating the purposes and advantages of the present invention, which, however, in no way limit the scope of the claims.

### Example 1

### Preparation of a medical plaster containing DicloNa 140 mg

The ingredients for the preparation of each medical plaster having dimensions of 140x100mm containing 140 mg of diclofenac sodium are:

| | | |
|---|---|---|
| **Ingredients** | **mg/plaster** | **%** |
| Diclofenac sodium | 140 | 7.25 |
| Eudragit^{®} NE40D (dry weight corresponding to 894.5 2236,38 mg of wet weight) | | 46.3 |
| Tributyl citrate (TBC) (Proviplast 2604 - Proviron) | 894.5 | 46.3 |
| Butylated hydroxyanisole (BHA) | 2.9 | 0.15 |

### Method

TBC and BHA were mixed under stirring and under vacuum for about 15 minutes, until they were completely dissolved. Diclofenac sodium was then added to the mixture, stirring under vacuum until complete dissolution, at a temperature not exceeding 25°C. Eudragit^{®} NE40D was then added, stirring rapidly for about 1 minute, then passing to medium stirring for 10 minutes and finally reducing the stirring rate to a minimum and maintaining it for 2 hours, at a temperature not higher than 25°C, continuing to operate under vacuum.

The mass was finally left to rest for 8 hours, continuously under vacuum.

The mass thus obtained was spread on a temporary *liner* made of siliconized polyester, and placed in a forced ventilation dryer for 5 minutes to dry, within a temperature gradient ranging from 65 to 115°C. The product thus obtained was then coupled by compression with the *backing* of 100% non-woven polyester and the temporary *liner* was subsequently replaced with the definitive *liner,* consisting of monosilicone paper, the plasters were then cut into the desired size (140x100mm) with the final primary packaging of the plasters thus obtained.

### Example 2

### Diclofenac release (Dissolution test)

The release of diclofenac from the medical plasters of the present invention was evaluated with the *Dissolution Test,* using as a comparison a commercial product that releases the active ingredient within 24 hours. The reference product is Flector^{®} (lot 1506031), which contains 180 mg of diclofenac hydroxyethylpyrrolidine salt corresponding to 140 mg of diclofenac sodium.

The active ingredient that exerts the pharmacological activity and whose release must be evaluated is diclofenac and not its salt, whatever it may be.

The *Dissolution Test* is performed as described in the European Pharmacopoeia (Ph. Eur. 5.0, 2.9.4), with the rotating cylinder method, using as *Dissolution Medium* a phosphate buffer in saline solution with a pH = 7.4 (PBS - Ph. Eur. 4.1.3), under the following operating conditions:

| | |
|---|---|
| Volume of *Dissolution Medium* | 900 ml |
| Temperature | 32°C ± 0.5°C |
| Rotation Rate | 100 rpm (revolutions/minute) |
| Sampling times | 1, 3, 6, 24 hours |
| Sampled quantity | 2 ml |

The analysis of the medium sampled was effected with HPLC equipment (Agilent 1100), according to what is known to skilled persons in the field.

Sample A is the plaster prepared according to Example 1, whereas Sample B is Flector^{®}.

A portion of 20 cm² (2x10 cm) was cut from each sample and, without removing the protective *liner,* was applied to the external surface of the cylinder with double-sided tape, so as to expose the surface containing the PSA matrix and the diclofenac dispersed therein to the *Dissolution Medium,* and in such a way that the greater axis of the portion of plaster corresponds perfectly to the circumference of the cylinder. When the temperature of the *Dissolution Medium* reached the expected value, the protective *liner* was removed, the sample was coated with a Cuprophan membrane, exceeding the sample size by at least 1 cm, and fixed with adhesive tape; the cylinder was finally immersed in the medium and immediately set in rotation at the prescribed speed. At the established intervals, a sample of 2 ml was taken from an intermediate area between the surface of the *Dissolution Medium* and the upper edge of the cylinder. The volume withdrawn was immediately replaced with an equal volume of fresh *Dissolution Medium.*

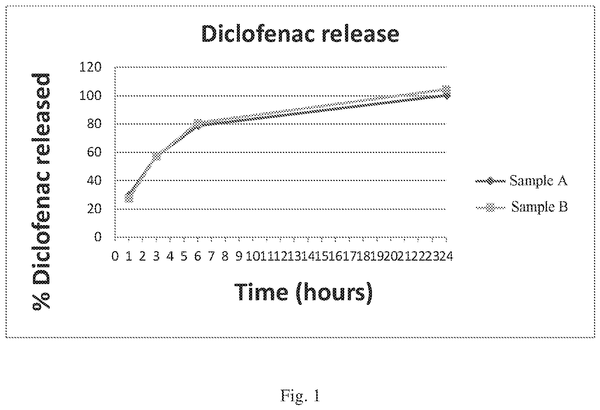

The dissolution values and the relative profile are shown respectively in the following Table 1 and in the Graph shown in Figure 1.

**Table 1**

| Time (hours) | Sample A | Sample B |
|---|---|---|
| | % Diclofenac release | % Diclofenac release |
| 1 | 29.66 | 27.35 |
| 3 | 57.38 | 57.34 |
| 6 | 78.67 | 80.75 |
| 24 | 100.24 | 104.66 |

It is evident that the diclofenac present in Sample A has a release profile that is absolutely comparable to the diclofenac contained in the Flector^{®} product (Sample B), a reference product among long-lasting medical plasters.

Sample A, specifically the medical plaster object of the invention, therefore releases the diclofenac from its sodium salt in a constant, continuous and gradual manner over 24 hours, which can effectively exert its pharmacological effect.

These data are quite surprising as they show that
- starting from a PSA matrix which is known to adequately release diclofenac diethylamine salt, but not diclofenac sodium salt and
- using diclofenac sodium salt which is known to be unsuitable for use in medical plasters
   by adding BHA to the PSA matrix formulation, i.e. a substance known for its antioxidant properties not necessary here, a medical plaster is obtained which
- releases the active principle in a constant, continuous, prolonged manner, at locally therapeutically active doses for a duration of 24 hours, and can therefore be replaced only once a day by the person using it;
- does not contain linear aliphatic amines or cyclic amines, and therefore does not cause exposure to the risk of toxic or irritative phenomena, and therefore can also be applied on sensitive or easily irritated skin;
- is industrially convenient, as the process for the preparation of the sodium salt is rapid, economical and does not involve the controlled disposal of processing waste.

## Claims

1. A medical plaster comprising
• a base layer,
• a "Pressure Sensitive Adhesive" (PSA) matrix,
• a protective coating layer,
wherein the PSA matrix comprises or consists of:
• a neutral copolymer based on ethyl acrylate and methyl methacrylate in a 2:1 ratio, in a concentration ranging from 40 to 49% by dry weight with respect to the dry weight of the matrix;
• a plasticizing agent selected from esters of citric acid, in a concentration ranging from 40 to 49% by weight with respect to the dry weight of the matrix;
• butylhydroxyanisole (BHA) in a concentration ranging from 0.10 to 0.20% by weight with respect to the dry weight of the matrix;
there being dispersed in said PSA matrix
• an active ingredient, which is diclofenac sodium, in a concentration ranging from 1 to 20% by weight with respect to the dry weight of the matrix.

2. The medical plaster according to claim 1, wherein the esters of citric acid are selected from triethyl citrate, acetyl triethyl citrate, acetyl tributyl citrate, tributyl citrate, preferably the ester is tributyl citrate.

3. The medical plaster according to one or more of the previous claims, wherein the PSA matrix comprises or consists of:
• a neutral copolymer based on ethyl acrylate and methyl methacrylate in a 2:1 ratio in an aqueous dispersion at 40% by weight, in a concentration ranging from 45 to 48% by dry weight with respect to the dry weight of the matrix;
• a plasticizing agent which is tributyl citrate, in a concentration ranging from 45 to 48% by weight with respect to the dry weight of the matrix;
• BHA in a concentration ranging from 0.13 to 0.18% by weight with respect to the dry weight of the matrix;
diclofenac sodium being dispersed in said PSA matrix, in a concentration ranging from 5 to 10% by weight with respect to the dry weight of the matrix.

4. The medical plaster according to one or more of the previous claims, wherein the PSA matrix comprises or consists of:
• a neutral copolymer based on ethyl acrylate and methyl methacrylate in a 2:1 ratio in an aqueous dispersion at 40% by weight, in a concentration equal to 46.3% by dry weight with respect to the dry weight of the matrix;
• tributyl citrate, in a concentration equal to 46.3% by weight with respect to the dry weight of the matrix;
• BHA in a concentration equal to 0.15% by weight with respect to the dry weight of the matrix;
diclofenac sodium being dispersed in said PSA matrix, in a concentration equal to 7.25% by weight with respect to the dry weight of the matrix.

5. The medical plaster according to one or more of the previous claims, wherein the base layer consists of a non-perforated 100% polyester non-woven fabric.

6. The medical plaster according to one or more of the previous claims, wherein the protective coating layer is a protective sheet of monosilicone paper.

7. A medical plaster according to one or more of the previous claims, for use in the once-a-day treatment of painful and inflammatory conditions affecting the musculoskeletal system, such as for example osteoarthritis, and also traumas such as sprains, muscle tears, bruises with intact skin.

8. A "Pressure Sensitive Adhesive" (PSA) matrix comprising or consisting of:
• a neutral copolymer based on ethyl acrylate and methyl methacrylate in a 2:1 ratio, in a concentration ranging from 40 to 49% by dry weight with respect to the dry weight of the matrix;
• a plasticizing agent selected from esters of citric acid, in a concentration ranging from 40 to 49% by weight with respect to the dry weight of the matrix;
• butylhydroxyanisole (BHA) in a concentration ranging from 0.10 to 0.20% by weight with respect to the dry weight of the matrix;
there being dispersed in said PSA matrix
• an active ingredient, which is diclofenac sodium, in a concentration ranging from 1 to 20% by weight with respect to the dry weight of the matrix.

9. The "Pressure Sensitive Adhesive" (PSA) matrix according to claim 8, wherein the esters are selected from triethyl citrate, acetyl triethyl citrate, acetyl tributyl citrate, tributyl citrate, preferably the ester is tributyl citrate.

10. The "Pressure Sensitive Adhesive" (PSA) matrix according to claim 8, comprising or consisting of:
• a neutral copolymer based on ethyl acrylate and methyl methacrylate in a 2:1 ratio in an aqueous dispersion at 40% by weight, in a concentration ranging from 45 to 48% by dry weight with respect to the dry weight of the matrix;
• a plasticizing agent which is tributyl citrate, in a concentration ranging from 45 to 48% by weight with respect to the dry weight of the matrix;
• BHA in a concentration ranging from 0.13 to 0.18% by weight with respect to the dry weight of the matrix;
diclofenac sodium being dispersed in said PSA matrix, in a concentration ranging from 5 to 10% by weight with respect to the dry weight of the matrix.

11. The "Pressure Sensitive Adhesive" (PSA) matrix according to claim 8, comprising or consisting of:
• a neutral copolymer based on ethyl acrylate and methyl methacrylate in a 2:1 ratio in an aqueous dispersion at 40% by weight, in a concentration equal to 46.3% by dry weight with respect to the dry weight of the matrix;
• tributyl citrate, in a concentration equal to 46.3% by weight with respect to the dry weight of the matrix;
• BHA in a concentration equal to 0.15% by weight with respect to the dry weight of the matrix;
diclofenac sodium being dispersed in said PSA matrix, in a concentration equal to 7.25% by weight with respect to the dry weight of the matrix.

## Patentansprüche

1. Medizinisches Pflaster, umfassend
• eine Basisschicht,
• eine "Pressure Sensitive Adhesive" (PSA)-Matrix,
• eine Schutzüberzugsschicht,
wobei die PSA-Matrix Folgendes umfasst oder daraus besteht:
• ein(em) neutrales(n) Copolymer auf Basis von Ethylacrylat und Methylmethacrylat in einem Verhältnis von 2:1 in einer Konzentration im Bereich von 40 bis 49 Trockengew.- %, bezogen auf das Trockengewicht der Matrix;
• ein(em) Weichmachermittel, ausgewählt aus Estern der Zitronensäure, in einer Konzentration im Bereich von 40 bis 49 Gew.-%, bezogen auf das Trockengewicht der Matrix;
• Butylhydroxyanisol (BHA) in einer Konzentration im Bereich von 0,10 bis 0,20 Gew.- %, bezogen auf das Trockengewicht der Matrix;
wobei es in der PSA-Matrix dispergiert ist;
• einen(m) Wirkstoff, der Diclofenac-Natrium ist, in einer Konzentration im Bereich von 1 bis 20 Gew.- %, bezogen auf das Trockengewicht der Matrix.

2. Medizinisches Pflaster nach Anspruch 1, wobei die Ester der Zitronensäure aus Triethylcitrat, Acetyltriethylcitrat, Acetyltributylcitrat, Tributylcitrat ausgewählt sind, vorzugsweise ist der Ester Tributylcitrat.

3. Medizinisches Pflaster nach einem oder mehreren der vorhergehenden Ansprüche, wobei die PSA-Matrix Folgendes umfasst oder daraus besteht:
• ein(em) neutrales(n) Copolymer auf Basis von Ethylacrylat und Methylmethacrylat in einem Verhältnis von 2:1 in einer wässrigen Dispersion zu 40 Gew.- % in einer Konzentration im Bereich von 45 bis 48 Trockengew.- % bezogen auf das Trockengewicht der Matrix;
• ein(em) Weichmachermittel, das Tributylcitrat ist, in einer Konzentration im Bereich von 45 bis 48 Gew.- %, bezogen auf das Trockengewicht der Matrix;
• BHA in einer Konzentration im Bereich von 0,13 bis 0,18 Gew.-%, bezogen auf das Trockengewicht der Matrix;
Diclofenac-Natrium, das in der PSA-Matrix dispergiert ist, in einer Konzentration im Bereich von 5 bis 10 Gew.- %, bezogen auf das Trockengewicht der Matrix.

4. Medizinisches Pflaster nach einem oder mehreren der vorhergehenden Ansprüche, wobei die PSA-Matrix Folgendes umfasst oder daraus besteht:
• ein(em) neutrales(n) Copolymer auf Basis von Ethylacrylat und Methylmethacrylat in einem Verhältnis von 2:1 in einer wässrigen Dispersion zu 40 Gew.- % in einer Konzentration gleich 46,3 Trockengew.- %, bezogen auf das Trockengewicht der Matrix;
• Tributylcitrat in einer Konzentration gleich 46,3 Gew.- %, bezogen auf das Trockengewicht der Matrix;
• BHA in einer Konzentration gleich 0,15 Gew.- %, bezogen auf das Trockengewicht der Matrix;
Diclofenac-Natrium, das in der PSA-Matrix dispergiert ist, in einer Konzentration gleich 7,25 Gew.- %, bezogen auf das Trockengewicht der Matrix.

5. Medizinisches Pflaster nach einem oder mehreren der vorhergehenden Ansprüche, wobei die Basisschicht aus einem nicht perforierten Vliesstoff aus 100 % Polyester besteht.

6. Medizinisches Pflaster nach einem oder mehreren der vorhergehenden Ansprüche, wobei die Schutzüberzugsschicht eine Schutzfolie aus Monosilikonpapier ist.

7. Medizinisches Pflaster nach einem oder mehreren der vorhergehenden Ansprüche zur Verwendung bei der einmal täglichen Behandlung von schmerzhaften und entzündlichen Erkrankungen, die den Bewegungsapparat betreffen, wie beispielsweise Arthrose, sowie Traumata wie Verstauchungen, Muskelrissen, Blutergüssen mit intakter Haut.

8. "Pressure Sensitive Adhesive" (PSA)-Matrix, umfassend oder bestehend aus:
• ein(em) neutrales(n) Copolymer auf Basis von Ethylacrylat und Methylmethacrylat in einem Verhältnis von 2:1 in einer Konzentration im Bereich von 40 bis 49 Trockengew.- %, bezogen auf das Trockengewicht der Matrix;
• ein(em) Weichmachermittel, ausgewählt aus Estern der Zitronensäure, in einer Konzentration im Bereich von 40 bis 49 Gew.-%, bezogen auf das Trockengewicht der Matrix;
• Butylhydroxyanisol (BHA) in einer Konzentration im Bereich von 0,10 bis 0,20 Gew.- %, bezogen auf das Trockengewicht der Matrix; wobei es in der PSA-Matrix dispergiert ist;
• einen(m) Wirkstoff, der Diclofenac-Natrium ist, in einer Konzentration im Bereich von 1 bis 20 Gew.- %, bezogen auf das Trockengewicht der Matrix.

9. "Pressure Sensitive Adhesive" (PSA)-Matrix nach Anspruch 8, wobei die Ester aus Triethylcitrat, Acetyltriethylcitrat, Acetyltributylcitrat, Tributylcitrat ausgewählt sind, vorzugsweise ist der Ester Tributylcitrat.

10. "Pressure Sensitive Adhesive" (PSA)-Matrix nach Anspruch 8, umfassend oder bestehend aus:
• ein(em) neutrales(n) Copolymer auf Basis von Ethylacrylat und Methylmethacrylat in einem Verhältnis von 2:1 in einer wässrigen Dispersion zu 40 Gew.- % in einer Konzentration im Bereich von 45 bis 48 Trockengew.- % bezogen auf das Trockengewicht der Matrix;
• ein(em) Weichmachermittel, das Tributylcitrat ist, in einer Konzentration im Bereich von 45 bis 48 Gew.- %, bezogen auf das Trockengewicht der Matrix;
• BHA in einer Konzentration im Bereich von 0,13 bis 0,18 Gew.-%, bezogen auf das Trockengewicht der Matrix;
Diclofenac-Natrium, das in der PSA-Matrix dispergiert ist, in einer Konzentration im Bereich von 5 bis 10 Gew.- %, bezogen auf das Trockengewicht der Matrix.

11. "Pressure Sensitive Adhesive" (PSA)-Matrix nach Anspruch 8, umfassend oder bestehend aus:
• ein(em) neutrales(n) Copolymer auf Basis von Ethylacrylat und Methylmethacrylat in einem Verhältnis von 2:1 in einer wässrigen Dispersion zu 40 Gew.- % in einer Konzentration gleich 46,3 Trockengew.- %, bezogen auf das Trockengewicht der Matrix;
• Tributylcitrat in einer Konzentration gleich 46,3 Gew.- %, bezogen auf das Trockengewicht der Matrix;
• BHA in einer Konzentration gleich 0,15 Gew.- %, bezogen auf das Trockengewicht der Matrix;
Diclofenac-Natrium, das in der PSA-Matrix dispergiert ist, in einer Konzentration gleich 7,25 Gew.- %, bezogen auf das Trockengewicht der Matrix.

## Revendications

1. Plâtre médical comprenant
• une couche de base,
• une matrice d'« adhésif sensible à la pression » (PSA),
• une couche de revêtement protecteur,
dans lequel la matrice de PSA comprend ou consiste en :
• un copolymère neutre à base d'acrylate d'éthyle et de méthacrylate de méthyle dans un rapport 2:1, dans une concentration comprise entre 40 et 49 % en poids sec par rapport au poids sec de la matrice ;
• un agent plastifiant choisi parmi les esters d'acide citrique, dans une concentration comprise entre 40 et 49 % en poids par rapport au poids sec de la matrice ;
• butylhydroxyanisole (BHA) dans une concentration comprise entre 0,10 et 0,20 % en poids par rapport au poids sec de la matrice ;
dispersés dans ladite matrice de PSA
• un ingrédient actif, qui est le diclofénac sodique, dans une concentration comprise entre 1 et 20 % en poids par rapport au poids sec de la matrice.

2. Plâtre médical selon la revendication 1, dans lequel les esters de l'acide citrique sont choisis parmi le citrate de triéthyle, le citrate d'acétyle de triéthyle, le citrate d'acétyle de tributyle, le citrate de tributyle, de préférence l'ester est le citrate de tributyle.

3. Plâtre médical selon une ou plusieurs des revendications précédentes, dans lequel la matrice de PSA comprend ou consiste en :
• un copolymère neutre à base d'acrylate d'éthyle et de méthacrylate de méthyle dans un rapport 2:1 dans une dispersion aqueuse à 40 % en poids, dans une concentration comprise entre 45 et 48 % en poids sec par rapport au poids sec de la matrice ;
• un agent plastifiant qui est le citrate de tributyle, dans une concentration comprise entre 45 et 48 % en poids par rapport au poids sec de la matrice ;
• BHA dans une concentration comprise entre 0,13 et 0,18 % en poids par rapport au poids sec de la matrice ;
le diclofénac sodique étant dispersé dans ladite matrice de PSA, dans une concentration comprise entre 5 et 10 % en poids par rapport au poids sec de la matrice.

4. Plâtre médical selon une ou plusieurs des revendications précédentes, dans lequel la matrice de PSA comprend ou consiste en :
• un copolymère neutre à base d'acrylate d'éthyle et de méthacrylate de méthyle dans un rapport 2:1 dans une dispersion aqueuse à 40 % en poids, dans une concentration égale à 46,3 % en poids sec par rapport au poids sec de la matrice ;
• citrate de tributyle, dans une concentration égale à 46,3 % en poids par rapport au poids sec de la matrice ;
• BHA dans une concentration égale à 0,15 % en poids par rapport au poids sec de la matrice ;
le diclofénac sodique étant dispersé dans ladite matrice de PSA, dans une concentration égale à 7,25 % en poids par rapport au poids sec de la matrice.

5. Plâtre médical selon l'une ou plusieurs des revendications précédentes, dans lequel la couche de base est constituée d'un tissu non tissé 100 % polyester non perforé.

6. Plâtre médical selon l'une ou plusieurs des revendications précédentes, dans lequel la couche protectrice est une feuille protectrice de papier monosilicone.

7. Plâtre médical selon l'une ou plusieurs des revendications précédentes, destiné à être utilisé dans le traitement uniquotidien des conditions douloureuses et inflammatoires affectant le système musculo-squelettique, comme par exemple l'arthrose, ainsi que des traumatismes tels que les entorses, les déchirures musculaires, les ecchymoses avec une peau intacte.

8. Matrice d'« adhésif sensible à la pression » (PSA) comprenant ou consistant en :
• un copolymère neutre à base d'acrylate d'éthyle et de méthacrylate de méthyle dans un rapport 2:1, dans une concentration comprise entre 40 et 49 % en poids sec par rapport au poids sec de la matrice ;
• un agent plastifiant choisi parmi les esters d'acide citrique, dans une concentration comprise entre 40 et 49 % en poids par rapport au poids sec de la matrice ;
• butylhydroxyanisole (BHA) dans une concentration comprise entre 0,10 et 0,20 % en poids par rapport au poids sec de la matrice ; dispersés dans ladite matrice de PSA
• un ingrédient actif, qui est le diclofénac sodique, dans une concentration comprise entre 1 et 20 % en poids par rapport au poids sec de la matrice.

9. Matrice d'« adhésif sensible à la pression » (PSA) selon la revendication 8, dans laquelle les esters sont choisis parmi le citrate de triéthyle, le citrate d'acétyle de triéthyle, le citrate d'acétyle de tributyle, le citrate de tributyle, de préférence l'ester est le citrate de tributyle.

10. Matrice d'« adhésif sensible à la pression » (PSA) selon la revendication 8, comprenant ou consistant en :
• un copolymère neutre à base d'acrylate d'éthyle et de méthacrylate de méthyle dans un rapport 2:1 dans une dispersion aqueuse à 40 % en poids, dans une concentration comprise entre 45 et 48 % en poids sec par rapport au poids sec de la matrice ;
• un agent plastifiant qui est le citrate de tributyle, dans une concentration comprise entre 45 et 48 % en poids par rapport au poids sec de la matrice ;
• BHA dans une concentration comprise entre 0,13 et 0,18 % en poids par rapport au poids sec de la matrice ;
le diclofénac sodique étant dispersé dans ladite matrice de PSA, dans une concentration comprise entre 5 et 10 % en poids par rapport au poids sec de la matrice.

11. Matrice d'« adhésif sensible à la pression » (PSA) selon la revendication 8, comprenant ou consistant en :
• un copolymère neutre à base d'acrylate d'éthyle et de méthacrylate de méthyle dans un rapport 2:1 dans une dispersion aqueuse à 40 % en poids, dans une concentration égale à 46,3 % en poids sec par rapport au poids sec de la matrice ;
• citrate de tributyle, dans une concentration égale à 46,3 % en poids par rapport au poids sec de la matrice ;
• BHA dans une concentration égale à 0,15 % en poids par rapport au poids sec de la matrice ;
le diclofénac sodique étant dispersé dans ladite matrice de PSA, dans une concentration égale à 7,25 % en poids par rapport au poids sec de la matrice.
